# EUROPEAN PATENT APPLICATION

(11) **EP 1 870 397 A1**
(43) Date of publication of application: **26.12.2007**
(21) Application number: 06380178.1
(22) Date of filing: 20.06.2006
(51) Int. Cl.: C07C 263/04, C07C 265/12, C07C 265/14

(54) **One-pot catalytic process for the synthesis of isocyanates and installations for the same**

(71) Applicant: REPSOL YPF S.A., 28046 Madrid (ES)
(72) Inventor: Serrano Fernández, Francisco Luis, 28935 Móstoles - Madrid (ES); Almena Muñoz, Beatriz, 28932 Móstoles - Madrid (ES); Padilla Polo, Ana, 28903 Getafe - Madrid (ES); Orejón Álvarez, Arancha, 43005 Tarragona (ES); Claver Cabrero, Carmen, 43893 Altafulla - Tarragona (ES); Castillón Miranda, Sergio, 43893 Altafulla - Tarragona (ES); Salagre Carnero, Pilar, 43202 Reus - Tarragona (ES)
(74) Representative: ABG Patentes, S.L.

(57) **Abstract**

One-pot process for the synthesis of isocyanates, polyisocyanates or mixtures thereof which comprises the steps of:
i. preparing a mixture comprising an amine, an alcohol, an oxygen containing gas, carbon monoxide, a metal macrocyclic complex catalyst over solid support and a solvent with high oxygen solubility;
ii. subjecting the resulting mixture to a first heating under pressure;
iii. cooling and depressurizing the mixture resulting from the previous step; and
iv. subjecting the mixture of the previous step to a second heating to separate out the isocyanate product from the mixture.

The present invention is also related to an installation to carry out the process.

## Description

### FIELD OF THE INVENTION

The present invention is related to the direct conversions of amines into isocyanates and installations to carry out said process.

### BACKGROUND OF THE INVENTION

Isocyanates are important chemicals. For example, the world production of isocyanates exceeded 5 megatons in 2001. Traditionally, isocyanates are manufactured on a commercial scale by reaction of phosgene with amines or amine salts. The reaction, however, has several serious drawbacks. Phosgene is an extremely toxic reagent and a stoichiometric amount of HCl is produced as a by-product. Furthermore, HCl causes serious corrosion, and stoichiometric amount of NaOH is required to neutralize the HCl, where the same amount of NaCl is formed. As restrictions upon the use of very toxic materials such as phosgene within the chemical industry have become more rigorously enforced, there has been increasing interest in developing alternative methods to phosgene in the synthesis of isocyanate.

As an alternative, the use of dimethyl carbonate or dimethyl sulphate as phosgene substitutes are relatively expensive for commercial applications (see M. Selva et. al., Tetrahedron Letters. 2002, 43 (7), 1217-1219; JP 20044262835; WO 98/56758; WO 99/47493).

Many others strategies for non-phosgene routes, including reductive carbonylation and oxidative carbonylation by using CO as carbonyl source, have been reported. One promising alternative approach that has been the subject of research in recent years involves the oxidative carbonylation of amines to carbamates in the presence of an alcohol, usually methanol, followed by catalytic decomposition of the carbamates to isocyanates.

The oxidative carbonylation of amines to carbamate ester catalyzed by a palladium group metal and iodide ions is disclosed in two closely related articles, namely J. Org. Chem. 1984, 49, 1458 and J. Chem. Soc., Chem. Commun, 1984, 399, both of Fukuoka and co-workers. They studied a liquid-solid carbonylation process in which solid Pd catalysts and solid iodide source are added to liquid alcohol at high pressure. Carbon monoxide (CO) and oxygen (O₂) are pressurized into the autoclave so as to become dissolved in the liquid reagents and react with the alcohol and the amine on the catalysts. While somewhat effective, this reaction necessarily involves catalyst recovery. Particularly, the solid Pd catalyst must be recovered from the carbamate product produced, which is not an easy task on industrial facilities. To recover the catalyst, complicated processes comprising distillation and extraction must be employed.

Alper and Hartstock (J. Chem. Soc., Chem. Commun. 1141, 1985) disclose catalytic systems including palladium chloride, copper chloride and hydrochloric acid to produce carbamates from amines. This Wacker-type catalytic system, consisting of PdCl₂-CuCl₂-HCl, is disclosed as being effective at mild conditions (1 atm and room temperature) in the oxidative carbonylation of amines to produce high yields of carbamate. In this system carbon monoxide (CO) and oxygen (O₂) are bubbled through an alcohol to which is added PdCl₂ and, finally, the amine. The mixture is stirred overnight, at room temperature and pressure, and filtered. The filtrate is subject to rotary evaporation. The resulting oil is treated with either diethyl ether or acetone and filtered, and concentration of the filtrate yields the carbamate ester. Further purification is carried out by thin-layer or column chromatography (silica gel). Thus, as in the process disclosed by Fukuoka and co-workers, a complex separation process is required.

Gupte and Chaudhari, Journal of Catalysis, 114, 246-258, 1988, studied the oxidative carbonylation of amines using a Pd/C-NaI catalytic system. Although effective at producing carbamates, this catalytic system uses a CO/O₂ molar ratio of 5/1, which is inside the flammability envelope.

US 4,976,679 (Okawa et al.) discloses a process for producing carbamates that comprises reacting a primary amine, an organic compound containing a hydroxyl group or groups (e.g. alcohol), carbon monoxide, and molecular oxygen in the presence of a catalytic system containing at least one member selected from copper and copper-containing compounds and at least one halogen selected from iodine, chlorine, and bromine. Thus, Okawa discloses that expensive palladium group metals are not necessary catalysts for the oxidative carbonylation of amines to carbamates.

US 2002/0183541 employ a Group VIII metal catalyst and/or copper-based catalyst with halide promoters to produce carbamate esters through heterogeneous oxidative carbonylation in a gas-solid carbonylation process. The carbamate produced remains on the catalyst surface and must be recovered through expensive extraction and distillation steps.

T. W. Leung, J. Chem. Soc. Chem. Comm., 3, 1992, 205-6 and US 5,194,660 describes a process for producing carbamates, using a homogeneous catalyst that comprises contacting a first reactant selected from primary amine component, secondary amine components, urea components and mixtures thereof; carbon monoxide; at least one oxygen-containing oxidizing agent, in the presence of catalyst composition comprising at least one metal macro-cyclic complex, preferably in the further presence of one iodine component. The macro-cyclic complex is selected from the group consisting of metal porphyrin or metal phthalocyanine including a metal selected from the metals of group IIIa to Va and group VIII of the Periodic Table and at least one iodine component is present in an amount effective to facilitate the formation of the carbamate. However, homogeneous catalyst systems, in general, suffer from drawbacks that are deterrent to their commercial use. The adverse characteristics include poor catalytic stability under reaction conditions, relatively high concentrations of catalyst in the reaction medium and low reaction rates to produce carbamates. Furthermore the catalyst must be recovered from the reaction mixture using expensive and costly recovering methods. Further, the severe reaction conditions, potential explosivity of the CO, O₂ blends, the organic hydroxyl component that must be used, the need of isolating the resulting carbamate and its degradation to the corresponding isocyanate on a second step have frustrated the use of this process in an industrial scale.

A. Bassoli et al., J. Mol. Catal. 1990, 60, 41 teaches the formation of ureas in good yields, with small amounts of carbamates and azo derivatives via the N,N-bis(salicylidene)ethylenediaminocobalt(II)-catalyzed oxidative carbonylation of aromatic primary amines in methanol.

E. Bolzacchini et al., J. Mol. Catal. A: Chemical, 111, 1996, 281-287, describes the N,N-bis(salicylidene)ethylenediaminocobalt(II)-catalyzed oxidative carbonylation of substituted aromatic primary amines in methanol to give blends of ureas, isocyanates, carbamates and azoderivatives. Said blends are unsuitable for the synthesis of commercial isocyanates. Furthermore the high reaction times needed (48 hours) precludes the industrial application.

US 03/0162995 describes a one-pot synthesis of isocyanates by reaction of amines with dimethyl carbonate and subsequent heating to obtain the isocyanate. Due to reaction conditions, the separation of the isocyanate product involves a complicated separation process which comprises water addition, further heating, filtration and a number of distillations in order to obtain the isocyanate, in impure form, which must be further purified.

Therefore, there is extensive literature regarding the production of isocyanates and polyisocyanates. The most commonly used procedure comprises the transformation of an amine into the corresponding carbamate in a first step, followed by the thermal decomposition of said carbamate to obtain the desired isocyanate or polyisocyanate. A large number of prior publications refer to one of these two steps.

For example, as mentioned above, US 5,194,660 discloses a method for the preparation of carbamates. However, the synthesis of the corresponding isocyanates is only suggested and only from the corresponding carbamates after isolation. Further, according to US 5,194,660 it is necessary to isolate the carbamate intermediate prior to its conversion into the desired isocyanate.

Only a few documents make reference or suggest the possibility of direct transformation of amines into the corresponding isocyanates. However, as in US 03/0162995 they usually require complicated separation steps.

In view of all of the above there is an existing need of providing an alternative cost effective and efficient

### SUMMARY OF THE INVENTION

It has now been surprisingly found an efficient, safe and cost effective one-pot process for the synthesis of isocyanate products.

The one-pot catalytic process disclosed herein satisfies the need in the art for an industrially viable oxidative carbonylation process capable of producing isocyanate products and at the same time does not require the isolation of intermediate carbamates.

Further, the process according to the present invention does not involve complicated separation steps. The isocyanate products are separated by means of direct distillation of the reaction mixture and, usually, the isocyanate product is obtained in essentially pure form.

Further, the present invention overcomes to a large extent the hazards associated with the direct reaction of carbon monoxide and oxygen in the presence of organic compounds by dissolving the reactant gases in a reaction solvent (e.g. halocarbons and/or oxygenated fluorinated hydrocarbons). Also, the one-pot process according to the present invention employs a heterogeneized cobalt complex catalyst, preferably with halide promoters, to produce isocyanates through heterogeneous oxidative carbonylation.

Therefore, according to a first aspect the present invention is related to a one-pot process for the synthesis of isocyanates, polyisocyanates or mixtures thereof which comprises the steps of:
- i.: preparing a mixture comprising an amine, an alcohol, an oxygen containing gas, carbon monoxide, a metal macrocyclic complex catalyst over solid support and a solvent with high oxygen solubility;
- ii.: subjecting the resulting mixture to a first heating under pressure;
- iii.: cooling and depressurizing the mixture resulting from the previous step; and
- iv.: subjecting the mixture of the previous step to a second heating to separate out the isocyanate product from the mixture.

According to a preferred embodiment, the solvent with high oxygen solubility is a halogenated organic molecule selected from aromatic halocarbons, perhalogenated alcohols, halogenated ethers, halogenated ketones, perfluorinated hydrocarbons, polymers of chlorotrifluoroethylene having the formula -(CF₂-CFCl)ₙ wherein n is between 2 and 10, or mixtures thereof.

According to a further embodiment, the alcohol is a perhalogenated alcohol and it is present in more than stoichiometric amounts so as to act as both, (co)solvent and reactant.

According to a second aspect, the present invention is related to an installation to carry out said process which comprises at least one reactor selected from slurry reactors with mechanical agitation, loop reactors, fixed bed reactors, fluidized bed reactors or combinations thereof.

These and other embodiments will be explained in the following detailed description of the invention.

### DETAILED DESCRIPTION OF THE INVENTION

As mentioned above, one aspect of the present invention refers to a one-pot process for the synthesis of isocyanates, polyisocyanates or mixtures thereof which comprises the steps of:
- i.: preparing a mixture comprising an amine, an alcohol, an oxygen containing gas, carbon monoxide, a metal macrocyclic complex catalyst over solid support and a solvent with high oxygen solubility;

- ii.: subjecting the resulting mixture to a first heating under pressure;
- iii.: cooling and depressurizing the mixture resulting from the previous step; and
- iv.: subjecting the mixture of the previous step to a second heating to separate out the isocyanate product from the mixture.

It is known that explosive concentrations of carbon monoxide in oxygen at 200°C and atmospheric pressure is 14,2-95,3 %, thus, the range is extremely broad. It is also known that dilution of the gaseous mixture with an inert gas like nitrogen scarcely changes the lower limit concentration. Further, variation of pressure between 1-200 atmospheres and temperatures between 0-200°C have a modest effect on the explosive range. Furthermore, even when these reactants are brought together in a ratio that, in the homogeneous conditions, would be outside the flammability envelope, the establishment of homogeneity from pure components involves at least a temporary passage through the flammability envelope. For these reasons, the explosion risks associated with the direct contacting of carbon monoxide and oxygen are not easily mitigated. Furthermore, unfortunately, carbon monoxide and oxygen are only slightly soluble in the alcohols used as solvents and reactants in the prior art.

To reduce the possibility of explosion, it is of course possible to also directly mix carbon monoxide and oxygen in a non-explosive ratio, outside of the flammability envelope. However, such ratios are far from the stoichiometric 2:1 molar ratio in which said reactants are consumed, thus requiring a large recycle of the non-limiting reactant. For example, when this mode of operation is used, it is preferable that oxygen and carbon monoxide are mixed continuously in a proportion of about 5% oxygen by volume and about 95% carbon monoxide by volume. Furthermore, while carbon monoxide/oxygen mixtures generally having less than about 5% oxygen by volume are non explosive, the establishment of this mixture from pure components always includes a transient period where at least some of the reactants are within the flammability envelope and pose an explosion hazard. Thus, the possibility for an explosive reaction mixture exists, regardless of the fact that the homogeneous reaction mixture is outside the flammability envelope.

It is known that mixing hazardous gases in the presence of fine sand that disperses these gases within interstitial voids surrounded by solid particles to absorb heat and/or radicals reduces the risk of explosions. Thus, commercial attempts to directly contact hydrogen and oxygen to form hydrogen peroxide have utilized this technique to avoid potential detonation upon passing from pure components through the flammability envelope and finally to a homogeneous, non-explosive mixture.

Taking this concept to its maximum logical extent, dissolution of gaseous reactants into a liquid solution disperses them even more finely. In this case, reactants are actually distributed within the reaction solvent at the molecular level. Thus, the use of a liquid solvent can essentially eliminate the explosion potential between dissolved reactants. Specifically, the surrounding liquid is a medium for absorbing the heat release and/or free radicals formed that would otherwise propagate an explosive reaction. In contrast, gaseous diluents do not provide nearly the same heat absorption capacity. Also, physically mixing with gaseous diluents generally results in transient or localized non-homogeneous reactants concentrations, in contrast to the dispersion at the molecular level that occurs upon dissolution of reactants gases.

An additional characteristic of the process of the present invention that overcomes the inherent hazards associated with explosive reaction mixtures is a gaseous environment above the liquid reaction mixture that is substantially free of feed components and oxygen. By substantially free is meant that, preferably, the sum of the concentrations of the feed components and oxygen in the gaseous environment above the reaction mixture is less than about 5% by volume, preferably less than about 2,5% by volume. The substantial absence of explosive species in the gaseous reaction environment above the liquid-phase reaction zone is maintained through directly dissolving the feed components and oxygen in the reaction liquid and continually sweeping, if necessary, any unreacted gases and contaminant gases existing in the liquid phase with a purge stream. This purge stream preferably comprises a noble gas (e.g. helium) or an otherwise inert gas selected from the group consisting of nitrogen, carbon dioxide, and mixtures thereof. Thus, the present invention avoids the need of any type of gaseous environment containing substantial quantities of oxygen.

The present invention uses solvents with high oxygen solubility, preferably, a halogenated organic molecule selected from aromatic halocarbons, perhalogenated alcohols, halogenated ethers, halogenated ketones, perfluorinated hydrocarbons, polymers of chlorotrifluoroethylene having the formula -(CF₂-CFCl)ₙ wherein n is between 2 and 10. The halocarbons and particularly fluorinated hydrocarbons are especially inert versus strong oxidizing agents, including oxygen, and dissolve gases readily. For example, it is known that solubility of oxygen in perfluoroalkanes is extremely high (Clark L. C. et al. Pure Appl. Chem., 1982, 54, 2383-2406 and Marrucho, I. M., Fluid Phase Equilibria, 222-223, 2004, 325-330). This is the reason why the present process can use lower pressures to achieve the same concentrations of the reactive gases in the liquid medium of reaction compared with the prior art processes that produce carbamates, and easily avoids gaseous environment containing any explosive mixture of reactants above the liquid reaction system. This is achieved by feeding reactants directly to the reaction medium and, if necessary, sweeping any unreacted components and contaminants above the liquid reaction system with an inert gas such as nitrogen from the reaction environment.

In order to carry out the oxidative carbonylation the presence of an alcohol is necessary since it acts as a reagent in the process of the invention. According to a preferred embodiment, said alcohol is a perhalogenated alcohol. As mentioned above, perhalogenated alcohols are good oxygen solvents. Therefore, perhalogenated alcohols can be present in the mixture of step i. in more than stoichiometric and at the same time act as a reactant and the solvent.

The feed and oxidizing agent may be dissolved in the reaction solvent in any order or fed simultaneously in separate streams to the reaction solvent. For example, it is possible to dissolve oxygen in the reaction solvent to saturation and subsequently contact the resulting oxygen saturated reaction solvent with the aromatic amine, the catalyst and carbon monoxide in a tubular mixer. Using the reaction solvents described later, the carbon monoxide solubility is generally affected to only a minor extent by the presence of oxygen in the reaction solvent.

In a further embodiment, risk of gas-phase contacting of reactants may also be eliminated by independently dissolving oxygen in the reaction solvent and carbon monoxide in the aromatic amine or polyamine. In this case, the dissolution steps may be carried out, for example, in separated stirred tanks before mixing all the reagents. Thus, it is not necessary to dissolve all the reagents in the same reaction vessel or at the same time. It is certainly possible that the reaction solvent containing previously dissolved reactants be passed through a fixed bed of catalyst or reacted in a slurry reactor. In the latter case, mechanical agitation (e.g. stirring, shaking, vibrating, etc.) can be used to effect the contacting between the catalyst and the reactants. It is also possible to use a liquid fluidized bed of catalyst, using a flow of gaseous carbon monoxide as described in *"*Perry's Chemical Engineers' Handbook, Sixth edition", 1984 pages 4-25, 4-26, 20-3 and 20-58 to 20-75.

According to one embodiment, the temperature during the first heating is comprised between 100 and 200°C, preferably between 120 and 180°C, and pressure is comprised between 5 and 100 bar, preferably between 20 and 70 bar. These conditions assure an effective oxidative carbonylation. The reaction time, which can vary depending on the reaction system employed, catalyst and other reaction conditions chosen. A typical reaction time is in the range of about one minute to about 3 hours.

According to a further embodiment, after the reaction mixture is cooled, the temperature during said second heating is comprised between 50 and 240° in order to separate the solvent by distillation and recover the isocyanate product.

According to a further embodiment, the separation of the solvent (step iv.) can be done through one, two or more, preferably two, consecutive distillation processes. Depending on the relative boiling points and nature of the isocyanate product and the solvents used, it is possible to directly obtain the isocyanate product by heating and subsequent distillation(s). Once the isocyanate product has been separated, without further treatment, the solvent may be recovered by a second subsequent distillation(s) of the mixture resulting from the first distillation(s). If the boiling point of the solvent is lower than the boiling point of the isocyanate product, it is possible to carry out a first distillation(s) to recover the solvent, followed by separation of the isocyanate product. Other combinations will be apparent to the skilled person depending on the relative boiling points and nature of the isocyanate product and the solvents used. In any case, the solvent recovered may be recirculated for use in further reactions.

According to a further embodiment, said first distillation(s) is carried out at a temperature comprised between 50 and 180°C and under a pressure comprised between 0,1 and 10 bar, and said second distillation(s) is carried out at a temperature comprised between 140 and 240°C and under a pressure comprised between 1 and 900 mbar. The condensation heat of the solvent from the first apparatus can be used for partially vaporizing solvent in the second apparatus. Recovered reaction solvent that is generated by this separation is normally most economically returned to the reaction solvent phase of the reactor for further reactions.

According to a further embodiment, prior to said second heating (step iv.), it is possible to add a solvent with high oxygen solubility to the reaction mixture.

The raw isocyanates obtained, can be purified, if desired, in a column with a top pressure of 1 to 950 mbar, preferably 5 to 250 mbar, with a bottom temperature of 60-250 °C and the pure isocyanates flow is withdrawn in liquid or gaseous form, preferably in a side-stream of the column.

It has been surprisingly found that the isocyanate products obtained following the process of the present invention are essentially pure without the need of further purification. Therefore, the process of the present invention as a whole is provides simple means for the industrial synthesis of isocyanate products.

The process according to the present invention can be carried out either batch-wise or in a continuous process by removing continuously the reaction mixture from the reaction system while continuously feeding the reactants into the reaction system. According to an embodiment of the invention, the process is a continuous process wherein space velocity is comprised between 20 and 40.000 h⁻¹ and the distilled solvent is recycled into the reaction.

Suitable amines and polyamines to be converted into isocyanates according to the invention include, substituted and unsubstituted aryl amines, for example, aniline, toluidine, 3,3'-dimethyl-4-4'-diphenylamine, phenylendiamines, toluendiamines, 2-4'- and 4,4'-methylendianiline, sulfonyldianilines, thiodianilines, diaminodiphenylmethanes and higher homologs polyaminopolyphenylmethanes, m-phenylenediamine, 1,5-naphthylenediamine and the like, and mixtures thereof; and substituted and unsubstituted aryl diamines or higher functionality polyamines like toluendiamines, diaminodiphenylmethanes or polyaminopolyphenylmethanes or any mixture thereof.

The reaction solvent used in the present invention is characterized in that it has a high carbon monoxide, amine feed and oxygen solubility. Reaction solvents comprise halocarbons and/or oxygenated fluorinated hydrocarbons. Halocarbons are preferably aryl halocarbons such as chlorobenzene and dichlorobenzene, fluorocarbons, chlorofluorocarbons, and hydrochlorofluorocarbons that are especially useful because their oxygen solubility limits represent, in some cases, higher oxygen concentrations on a mole/litter base than found in air. In fact, the oxygen solvency capacity of these materials has led to investigation of their use as blood substitutes. In terms of oxygen solubility, reaction solvents capable of dissolving greater than about 50 ml of oxygen per 100 ml of reaction solvent at 1 atmosphere are preferred. In most cases the solubility of other gases (e.g. carbon monoxide) within the reaction solvent is hardly affected by the degree of saturation of the reaction solvent with oxygen. Preferred reaction solvents are the completely fluorine-substituted C₅-C₈ hydrocarbons (e.g. perfluorohexane). These liquids are available under various trade names, such as 3M^{™} Performance Fluids (Minneapolis, Minn). Other preferred reaction solvents are the polymers of chlorotrifluoroethylene having the formula -(CF₂-CFCl)ₙ with n varying from 2 to about 10, such as the grade 0.8 of Halocarbon Product Corporation of USA. Those halocarbons can be added at the reaction mixture or at the outlet of the reactor to facilitate the work up of the mixture or to facilitate its cooling.

The reaction solvents also include oxygenated fluorinated hydrocarbons (e.g. fluorinated alcohols and its blends with fluorinated ethers and/or ketones), where at least one alkyl hydrogen of the homologous oxygenated is substituted for fluorine. Preferred oxygenated fluorinated hydrocarbons are 2,2,2-trifluoroethanol, 2,2,3,3-tetrafluoro-1-propanol, 1,1,1,3,3,3-hexafluoropropan-2-ol, nonafluoro tertbutanol and fluorophenols. Preferred fluorinated ether is nonafluorobutyl methyl ether. Preferred fluorinated ketone is hexafluoroacetone.

The metal of the catalyst according to the present invention preferably is a metal selected from the metals of Group VIII and more preferably the metal is cobalt. According to one embodiment of the present invention, the metal macrocyclic complex catalyst is selected a cobalt porphyrin of formula I 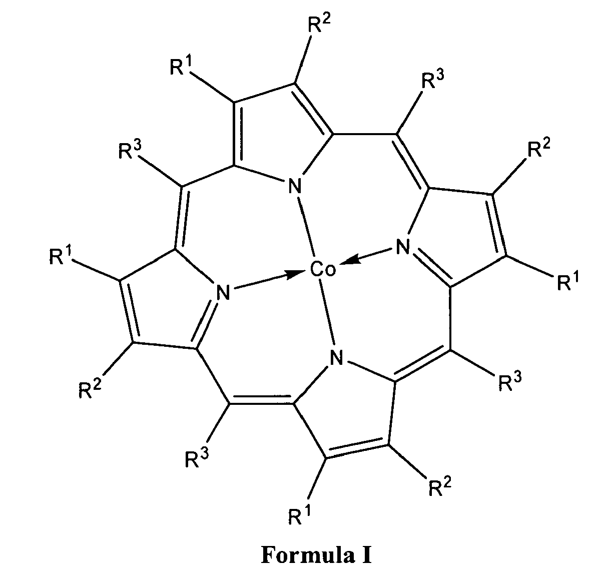 wherein
R¹ and R² are each independently selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl; or
R¹ and R² together form a substituted or unsubstituted cycloalkyl, substituted or unsubstituted cycloalkenyl, substituted or unsubstituted cycloalkinyl, substituted or unsubstituted aryl, substituted or unsubstituted heterocyclyl;
R³ is selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl;
or cobalt a phthalocyanine of formula II 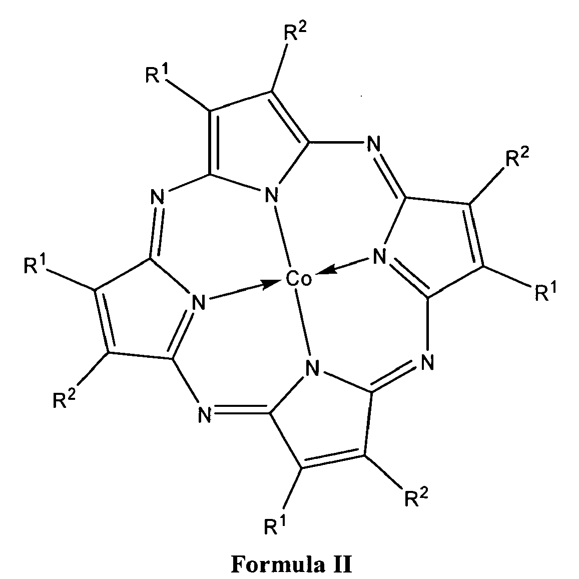 wherein
R¹ and R² have the same meaning as in formula I;
or a cobalt Shiff base ligand of formula III 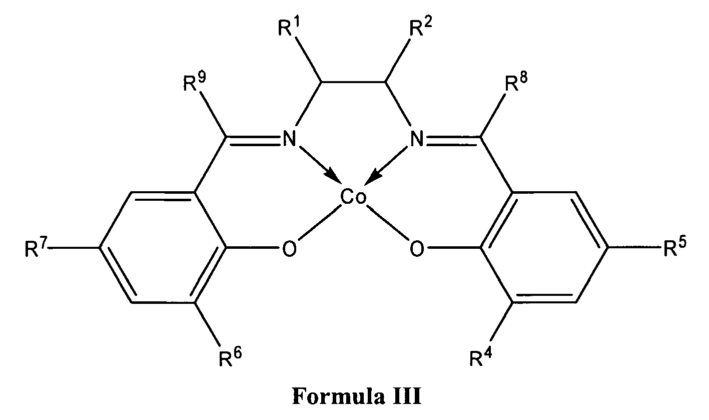 wherein
R¹ and R² have the same meaning as in formula I;
R⁴, R⁵, R⁶ and R⁷ are each independently selected from hydrogen, cyano, substituted or unsubstituted linear alkyl, substituted or unsubstituted branched alkyl, substituted or unsubstituted cycloalkyl, substituted or unsubstituted alkenyl, substituted or unsubstituted alkinyl, substituted or unsubstituted aryl, substituted or unsubstituted heterocyclyl;
R⁹ and R⁸ are each independently selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl;
or mixtures thereof.

The present invention also relies on heterogeneous reaction chemistry. Catalyst supports are preferred because they provide high surface area to disperse active catalyst components and they immobilize the active catalyst components. When carbon monoxide, oxygen and the amine react on the catalyst surface, the reaction products migrate into the organic phase, from which isocyanate products can be easily recovered by conventional distillation. Useful supports are well known in the art and may include, by way of non-limiting example, activated carbon and/or clay, i.e. Montmorillonite; polymer supports such as poly(styrene-divinylbenzene), polystyrene, and polyimide; mesoporous materials such as zeolite, MCM-41, ZSM-5, HZSM-5, ammonium ZSM-5 and SBA-15; and metal oxides such as gamma-Al₂O₃, SiO₂ and TiO₂, and MgO, silica or inorganic refractory metal oxides.

Said catalysts can be prepared by known methods. For example, Co-clay can be prepared by anchoring the salen ligand in the interlayers of Montmorillonite and subsequent complexation with cobalt acetate by the method described by Choudhari et al. (J. Chem. Soc., Chem. Commun., 1987, 1505); the immobilization of Co-salen derivatives on mesoporous silica gel and MCM-41 by using grafting reactions, for example, according to I. C. Chisem et al., Chem. Commun. 1998, 1949; P. Sutra et al. Chem. Commun. 1996, p. 2485; X.-G Zhou et al. Chem. Commun. 1999, 1789 or R.J.P. Corriu et al., J. Mater. Chem., 2002, 12, 1355-1362 o by the method described in US 2005/0131252.

Surprisingly, the heterogeneous reaction, where the metal macrocyclic complex catalyst is fixed to a solid support, overcomes the difficulties of homogeneous systems associated with the lost of catalyst, limited catalyst solubility and stability and/or metal precipitation. This is another fundamental advantage of this process versus prior art processes that uses homogeneous catalysts.

The use of metal macrocyclic complex catalyst permits an easy separation from the reaction mixture.

Furthermore, the process of the present invention can be carried out in the absence or in the presence of a promoter. According to one embodiment, the halide promoter can be selected from alkali metal halides, alkaline earth metal halides, onium halides, compounds capable of forming onium halides at the contacting conditions, oxo acids of halogen atom and their salts, organic halides and halogen molecules. Those compounds containing iodine are particularly preferred. These include KI, NaI, LiI, CsI, tetrabutylammonium iodine, tetraheptyl ammonium iodide, iodous acid, iodine and the like.

As mentioned above, an aspect of the present invention refers to an installation to carry out the process of the present invention characterised in that it comprises at least one slurry reactor with mechanical agitation, at least one loop reactor, at least one fixed bed reactor, at least one fluidized bed reactor or combinations thereof.

The process of this invention can be conducted in a reactor or in a cascade of two o more reactors of any conventional design suitable for liquid phase process. These designs include fixed-bed, transport bed, fluidized bed, moving bed, shell and tube or trickle bed reactors. Preferred reactor types are continuously stirred tank reactors, bubble columns or loop reactors. According to an embodiment of the present invention, the process of this invention is conducted in a cascade of 2 to 10, preferably 2 to 7, most preferably 2 to 5 reactors, and oxygen or oxygen containing gas and carbon monoxide added in several portions to several reactors.

Alternatively, the process of this invention can be conducted in a baffled tank reactor or in plug flow reactor and oxygen or oxygen-containing gas and carbon monoxide added in several portions at several feed points to the reactor, that is, at several reactor stages.

In the above definition of the process and compounds and in the description and claims the following terms have the meaning indicated:
"One-pot" refers to processes which do not involve isolation of any intermediates prior to recovery of the final product, regardless of the number of steps required.
"Essentially pure" refers to compounds which require no purification prior to further use. Typically, a product having purity higher than 98% w/w is considered essentially pure. However, for some applications isocyanate products having purity higher than 95% w/w are also considered essentially pure.
"perhalogenated" refers to organic molecules in which hydrogen is substituted in two or more positions by a halogen group, preferably in all positions. For example, perhalogenated alkyl groups are those in which at least two hydrogen atoms are substituted by a halogen atom, e.g.
"halogenated" refers to organic molecules in which hydrogen is substituted in at least one position by a halogen group. Therefore, perhalogenated molecules are comprised within the group of halogenated molecules.
"isocyanate product" refers to the products obtained by putting into practice the process according to the present invention, including isocyanates and polyisocyanates.
"Alkyl" refers to a straight or branched hydrocarbon chain radical consisting of carbon and hydrogen atoms, containing no saturation, having 1-12, preferably one to eight carbon atoms, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as halo, hydroxy, alkoxy, OPr, OBn, OBz, carboxy, cyano, carbonyl, acyl, alkoxycarbonyl, amino, imino, nitro, mercapto and alkylthio.
"Alkoxy" refers to a radical of the formula -ORa where Ra is an alkyl radical as defined above, e. g., methoxy, ethoxy, propoxy, etc.
"Aryloxy" refers to a radical of formula -ORb wherein Rb is an aryl radical as defined below.
"Amino" refers to a radical of the formula -NH₂, -NHRa, -NRaRb.
"Aryl" refers to an aromatic hydrocarbon radical such as phenyl, naphthyl or anthracyl. The aryl radical may be optionally substituted by one or more substituents such as hydroxy, mercapto, halo, alkyl, phenyl, alkoxy, haloalkyl, nitro, cyano, dialkylamino, aminoalkyl, acyl and alkoxycarbonyl, as defined herein.
"Aralkyl" refers to an aryl group linked to an alkyl group such as benzyl and phenethyl.
"Cycloalkyl" refers to a saturated carbocyclic ring having from 3 to 8 carbon atoms.
"Heterocyclyl" refers to a stable 3- to 15- membered ring which consists of carbon atoms and from one to five heteroatoms selected from the group consisting of nitrogen, oxygen, and sulphur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.
"Complex" refers to a molecule which is formed by two components: a donor and an acceptor. Bonding between both components to form the complex is possible because the donor may donate an unshared pair of electrons or electrons on π orbitals, which the acceptor can accommodate. In a complex more than one donor and/or more than one acceptor are possible. Also, in the same "complex" one donor may be bonded to more than one acceptor and vice versa. Besides the donor-acceptor interactions described above, other types of bonding know to the skilled person, such as covalent bonding, may exist between the donor and the acceptor.

References herein to substituted groups in the compounds of the present invention refer to the specified moiety that may be substituted at one or more available positions by one or more suitable groups, e. g., halogen such as fluoro, chloro, bromo and iodo; cyano; hydroxyl ; nitro ; azido ; alkanoyl such as a C1-12 alkanoyl group such as acyl and the like; carboxamido ; alkyl groups including those groups having 1 to about 12 carbon atoms or from 1 to about 6 carbon atoms and more preferably 1-3 carbon atoms; alkenyl and alkynyl groups including groups having one or more unsaturated linkages and from 2 to about 12 carbon or from 2 to about 6 carbon atoms; alkoxy groups having one or more oxygen linkages and from 1 to about 12 carbon atoms or 1 to about 6 carbon atoms ; aryloxy such as phenoxy; alkylthio groups including those moieties having one or more thioether linkages and from 1 to about 12 carbon atoms or from 1 to about 6 carbon atoms; alkylsulfinyl groups including those moieties having one or more sulfinyl linkages and from 1 to about 12 carbon atoms or from 1 to about 6 carbon atoms; alkylsulfonyl groups including those moieties having one or more sulfonyl linkages and from 1 to about 12 carbon atoms or from 1 to about 6 carbon atoms; aminoalkyl groups such as groups having one or more N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

Unless otherwise stated, the compounds obtainable by the process of the invention are also meant to include compounds which differ only in the presence of one or more isotopically enriched atoms. For example, compounds obtainable by the process of the invention having the same structures except for the replacement of a hydrogen by a deuterium or tritium, or the replacement of a carbon by a ¹³C- or ¹⁴C-enriched carbon or ¹⁵N-enriched nitrogen are within the scope of this invention.

The invention will be further illustrated by means of examples, which should not be interpreted as limiting the scope of the claims.

### EXAMPLES

### Example 1: general procedure for the formation of a Schiff base-type ligand catalyst

An aldehyde is refluxed in the presence of the corresponding diamine (2:1 molar ratio) in ethanol 95%. Filtration and further washing with absolute ethanol afforded the essentially pure Schiff base-type ligand.

The Schiff base-type ligand obtained above is refluxed in an alcoholic solution together with an aqueous solution of Co(OAC)₂·4H₂O under inert atmosphere. Although a crystalline precipitate immediate appeared, refluxing continues for about an hour. The precipitate obtained is filtered under vacuum, washed with water, ethanol and ether, and then dried under vacuum.

### Example 2: immobilization of the catalyst to a solid support

N,N'-(bis(3,5-di-tertbutyl-salicylaldehyde)ethylenediamino)cobalt (II) [Co-tBu-Salen], 1,9 g, prepared following the procedure of Example 1, is added to 125 ml of 2-propanol with stirring, and the mixture heated to 70°C. Powdered silica (5 g, Grace Davison XPO 2407, specific surface area 250 m²/g) is added to the solution and temperature and the stirring are maintained for a further 5 hours. The suspension is filtered, the solid residue is washed with 25 ml dichloromethane anhydrous, twice, and finally dried under vacuum for 5 hours.

### Example 3: comparative

A mixture of the aniline (13,2 mmol), the cobalt complex [Co-tBu-Salen] (0,25 mmol) prepared following Example 1, NaI (2,72 mmol) and 1-butanol (20 ml) is charged in a 100 ml autoclave. The autoclave is flushed with carbon monoxide and oxygen in a volumetric ratio of 19/1 to a total pressure of 52,7 bar. The temperature is increased to 110°C. The reaction is maintained under constant vigorous stirring for three hours. After 3 hours the reactor is cooled to room temperature, depressurised and 97,8 g of 1,2-Dichlorobenzene (DCB) is charged in the reactor. Then the temperature is increased to 180°C under atmospheric pressure. No 1-butanol is recovered by condensation of the vapours. Analysis of the reaction residue confirms the existence of urea derivatives but no traces of phenylisocyanate may be found.

### Example 4: Synthesis of phenylisocyanate

In a 1000 ml high-pressure stirred autoclave 15,5 g of aniline, 7,6 g of the [Co-tBu-Salen]/Silica catalyst of Example 2, 1,78 g of NaI and 488 g of 2,2,2-trifluoroethanol (TFE) are charged. The autoclave is pressurized with carbon monoxide and oxygen in a volumetric ratio of 19/1. The temperature is increased to 120°C and the total pressure is maintained constant at 40 bars. The reaction is maintained under constant vigorous stirring for three hours. After 3 hours the reactor is cooled to room temperature, depressurised and 97,8 g of 1,2-dichlorobenzene (DCB) is charged in the reactor. Then the temperature is increased to 180°C under atmospheric pressure. The vapours of TFE are separated, condensed, and reused in the following example. In the bottom of the reactor a mixture of DCB and phenylisocyanate is obtained. From this mixture pheylisocyanate is recovered by distillation with 99% w/w purity.
Yield of the intermediate carbamate was 94 % and yield of the final isocyanate was 54,6% (51,3% overall yield).

### Example 5: Synthesis of 2,4-toluendiisocyanate

In a 1000 ml high-pressure stirred autoclave 10 g of 2,4-toluendiamine (TDA), 7,6 g of [Co-tBu-Salen]/Silica catalyst of Example 2, 1,7 g of NaI and 484 g of 2,2,2-trifluoroethanol (TFE) are charged. The autoclave is pressurized with carbon monoxide and oxygen in a volumetric ratio of 19/1. The temperature is increased up to 120°C and the total pressure is maintained constant at 40 bars. The reaction is maintained under constant vigorous stirring for three hours. After 3 hours the reactor is cooled to room temperature, depressurised and 97,8 g of 1,2-dichlorobenzene (DCB) is charged in the reactor. Then the temperature is increased to 180°C under atmospheric pressure. The vapours of TFE are separated, condensed, and reused in the following example. In the bottom of the reactor a mixture of DCB and 2,4-toluendiisocyanate is obtained. From this mixture 2,4-toluendiisocyanate is recovered by distillation with 99% w/w purity.
Yield of the intermediate carbamate was 84,1 % and yield of the final isocyanate was 83% (69,8% overall yield).

## Claims

1. One-pot process for the synthesis of isocyanates, polyisocyanates or mixtures thereof which comprises the steps of:
i. preparing a mixture comprising an amine, an alcohol, an oxygen containing gas, carbon monoxide, a metal macrocyclic complex catalyst over solid support and a solvent with high oxygen solubility;
ii. subjecting the resulting mixture to a first heating under pressure;
iii. cooling and depressurizing the mixture resulting from the previous step; and
iv. subjecting the mixture of the previous step to a second heating to separate out the isocyanate product from the mixture.

2. One-pot process according to claim 1, **characterised in that** the solvent with high oxygen solubility is a halogenated organic molecule selected from aromatic halocarbons, perhalogenated alcohols, halogenated ethers, halogenated ketones, perfluorinated hydrocarbons, polymers of chlorotrifluoroethylene having the formula -(CF₂-CFCl)ₙ wherein n is between 2 and 10, or mixtures thereof.

3. One-pot process according to claim 1, **characterised in that** the alcohol is a perhalogenated alcohol.

4. One-pot process according to claim 3, **characterised in that** the perhalogenated alcohol is present in more than stoichiometric amounts so as to act as both, (co)solvent and reactant.

5. One-pot process according to any of the previous claims, **characterised in that** the temperature in said first heating is comprised between 100 and 200°C, preferably between 120 and 180°C, and pressure is comprised between 5 and 100 bar, preferably between 20 and 70 bar.

6. One-pot process according to any of the previous claims, **characterised in that** the temperature in said second heating is comprised between 50 and 240°C.

7. One-pot process according to any of the previous claims, **characterised in that** a solvent with high oxygen solubility is added prior to said second heating.

8. One-pot process according to any of the previous claims, **characterised in that** step iv. comprises at least one distillation to remove solvents and at least one distillation to separate the isocyanate product.

9. One-pot process according to claim 8, **characterised in that** said distillations are independently selected from distillations under pressure, distillations under vacuum or distillations at atmospheric pressure.

10. One-pot process according to any of claims 8 or 9, **characterised in that** said first distillation is carried out at a temperature comprised between 50 and 180°C and under a pressure comprised between 0,1 and 10 bar, and said second distillation is carried out at a temperature comprised between 140 and 240°C and under a pressure comprised between 1 and 900 mbar.

11. One-pot process according to any of claims 8-10, **characterised in that** the distilled solvents are recycled for further reactions.

12. One-pot process according to any of claims 1-11, **characterised in that** it is a continuous process.

13. One-pot process according to claim 12, **characterised in that** space velocity is comprised between 20 and 40.000 h⁻¹.

14. One-pot process according to any of claims 12 or 13, **characterised in that** the distilled solvent is recycled into the reaction.

15. One-pot process according to any of the previous claims, **characterised in that** the oxygen concentration in the gaseous phase is less than 5%, preferably less than 2,5%.

16. One-pot process according to any of the previous claims, **characterised in that** the metal macrocyclic complex catalyst is selected from metals of Group VIII and more preferably is a cobalt porphyrin of formula I 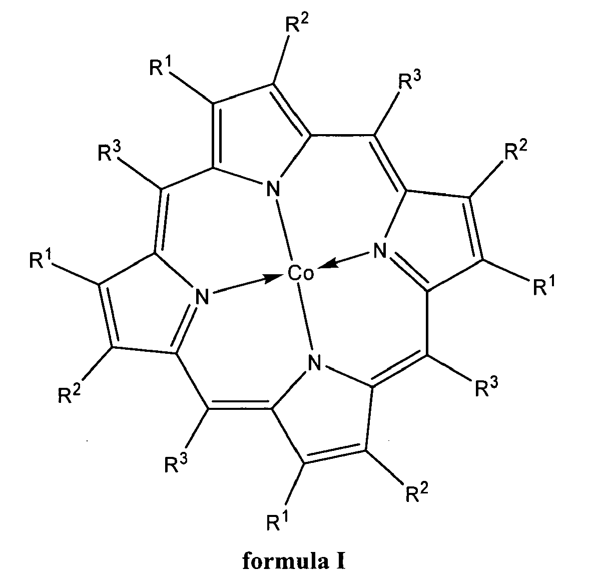 wherein
R¹ and R² are each independently selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl; or
R¹ and R² together form a substituted or unsubstituted cycloalkyl, substituted or unsubstituted cycloalkenyl, substituted or unsubstituted cycloalkinyl, substituted or unsubstituted aryl, substituted or unsubstituted heterocyclyl;
R³ is selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl;
or a cobalt phthalocyanine of formula II 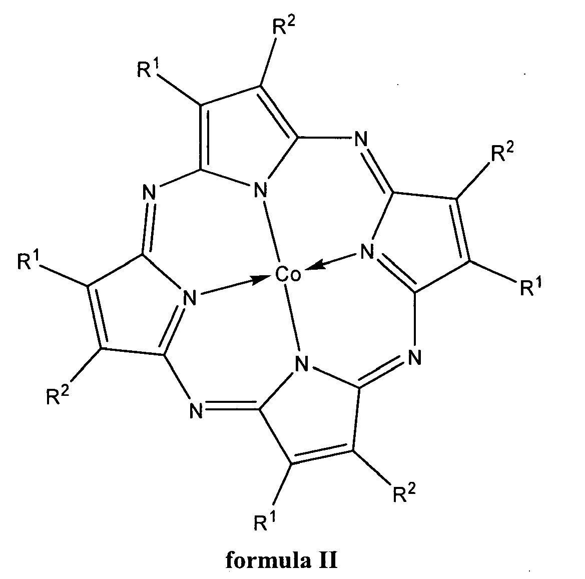 wherein
R¹ and R² have the same meaning as in formula I;
or a cobalt Shiff base ligand of formula III 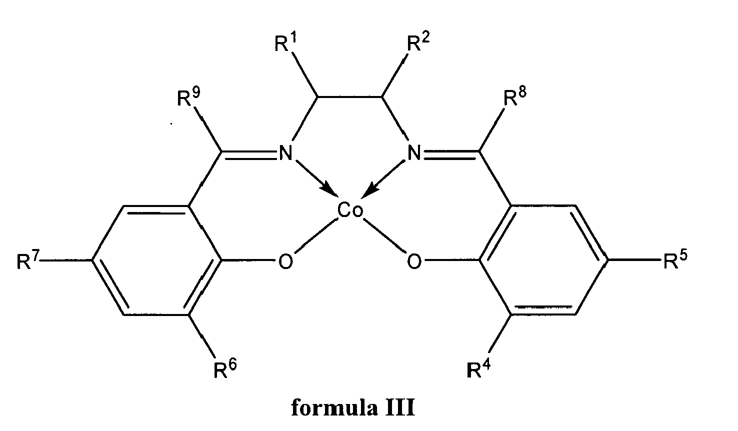 wherein
R¹ and R² have the same meaning as in formula I;
R⁴, R⁵, R⁶ and R⁷ are each independently selected from hydrogen, cyano, substituted or unsubstituted linear alkyl, substituted or unsubstituted branched alkyl, substituted or unsubstituted cycloalkyl, substituted or unsubstituted alkenyl, substituted or unsubstituted alkinyl, substituted or unsubstituted aryl, substituted or unsubstituted heterocyclyl;
R⁹ and R⁸ are each independently selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl;
or mixtures thereof.

17. One-pot process according to any of the previous claims, **characterised in that** the solid support is selected from silica, inorganic refractory metal oxides, zeolites, carbon or polymers or mixtures thereof.

18. One-pot process according to any of the previous claims, **characterised in that** the isocyanate product obtained is essentially pure.

19. One-pot process according to any of the previous claims, **characterised in that** the amine is selected from substituted or unsubstituted aryl amines, substituted or unsubstituted aryl diamines, polyaminopolyphenylmethanes or mixtures thereof.

20. One-pot process according to claim 19, **characterised in that** the amine is selected from the group consisting of toluenediamines, diaminodiphenylmethanes or mixtures thereof.

21. One pot process according to claim 1, **characterised in that** a halide promoter selected from alkali metal halides, alkaline earth metal halides, onium halides, compounds capable of forming onium halides at the contacting conditions, oxo acids of halogen atom and their salts, organic halides and halogen molecules or mixtures thereof, is added prior to said first heating under pressure.

22. Installation to carry out the process of any of the preceding claims **characterised in that** it comprises at least one reactor selected from slurry reactors with mechanical agitation, loop reactors, fixed bed reactors, fluidized bed reactors or combinations thereof.

23. Installation according to claim 22, **characterised in that** said installation comprises 2 to 10, preferably 2 to 7, more preferably 2 to 5 reactors wherein carbon monoxide and the oxygen containing gas are added to at least one of said reactor in different portions.

24. Installation according to any of claims 22-23, **characterised in that** the oxygen containing gas is added as a solution of a solvent with high oxygen solubility.
